# EUROPÄISCHE PATENTSCHRIFT

(11) **EP 0 621 051 B1**
(45) Veröffentlichungstag und Bekanntmachung des Hinweises auf die Patenterteilung: **22.08.2001**
(21) Anmeldenummer: 94105529.5
(22) Anmeldetag: 09.04.1994
(51) Int. Cl.: A61M 16/08, A61M 16/12

(54) **Gerät zur kontrollierten Zudosierung von NO zur Atemluft von Patienten**
Apparatus for the controlled dosage of NO to a patient's air supply
Appareil de contrôle du dosage en NO de l'air aspiré pour un patient

(30) Priorität: 17.04.1993 DE 4312431; 29.07.1993 DE 4325319
(43) Veröffentlichungstag der Anmeldung: 26.10.1994
(73) Patentinhaber: MESSER GRIESHEIM AUSTRIA Ges.m.b.H., 2352 Gumpoldskirchen (AT)
(72) Erfinder: Krebs, Christian, A-1120 Wien (AT)
(74) Vertreter: Berdux, Klaus, Dipl.-Ing.

(56) Entgegenhaltungen:
- WO-A-90/04425
- WO-A-91/14476
- WO-A-92/10228
- WO-A-92/11052

## Beschreibung

Die Erfindung betrifft ein Gerät zur kontrollierten Zudosierung von NO zur Atemluft und Analyse der inspiratorischen sowie exspiratorischen Atemluft von Patienten.

ARDS (adult respiratory distress syndrome) ist ein schweres Lungenversagen des Erwachsenen, bei dem neben einer anhaltenden schweren Störung des Gasaustausches der Lunge ein Bluthochdruck im Lungenkreislauf erfolgt. Dieser Hochdruck führt zu einer schweren Rechts-Herz-Belastung und in Konsequenz zu einem Rechts-Herz-Versagen. Außer bei ARDS kann Hochdruck im Lungenkreislauf auch auftreten bei frühgeborenen Kindern und bei bestimmten Herzmißbildungen. Der Hochdruck im Lungenkreislauf kann an sich gesenkt werden durch Verabreichung blutdrucksenkender Mittel. Diese wirken jedoch auf den gesamten Blutkreislauf. Da der Blutdruck bei Patienten mit ARDS in der Regel schon zu niedrig ist, können solche blutdrucksenkenden Mittel daher nur mit großen Einschränkungen verabreicht werden.

Seit einigen Jahren ist es bekannt, daß durch Zudosierung von NO zur Atemluft von Patienten der Hochdruck im Lungenkreislauf gezielt gesenkt werden kann. Das NO wirkt gefäßerweiternd und führt infolge der Vergrößerung des Gefäßquerschnittes zu einer Senkung des Blutdruckes im Lungenkreislauf. Schwierigkeiten bereitet die Behandlung mit NO insofern, als eine störungsfreie Behandlung mit NO über einen Zeitraum von mehreren Wochen sichergestellt sein muß. Eine weitere Schwierigkeit kann dadurch entstehen, daß sich in der Lunge des Patienten aus NO teilweise giftiges NO₂ bilden kann.

WO-A-90 04425 beschreibt eine Vorrichtung zur Entfeuchtung von ausgeatmetem Gas von einem Patienten während einer medizinischen Behandlung wie Anästhesie. Das ausgeatmete Gas wird über den Entfeuchter einem Analysegerät zugeführt, das nicht näher spezifiziert wird.

WO-A-92 10228 beschreibt ein System zur Zudosierung von NO in die Atemluft eines Patienten. Die Zumischung von NO erfolgt über einen Gasballon (gas mixture bag). Ein Inhalationsgerät für NO-haltiges Gas und/oder eine inhalierbare Substanz wird beschrieben, das ohne ein Beatmungsgerät arbeitet und die Luft von der Außenluft bezieht.

Der Erfindung liegt die Aufgabe zugrunde, ein Gerät zur kontrollierten Zudosierung von NO zur Atemluft von Patienten zu schaffen, welches einen sicheren Dauerbetrieb gewährleistet und sehr schnell auf unzulässige Änderungen der Konzentration von NO und gegebenenfalls NO₂ in der Atemluft des Patienten reagiert. Die Aufgabe wird gelöst durch ein Gerät mit den in Anspruch 1 beschriebenen Merkmalen.

Vorteilhafte Weiterbildungen der Erfindung sind in den Unteransprüchen angegeben.

Für das erfindungsgemäße Gerät geeignete Analysatoren arbeiten beispielsweise nach dem Infrarot-Absorptionsmeßprinzip, dem Meßprinzip der elektrochemischen Sensoren oder dem Chemilumineszenzdetektor-Prinzip. Der Chemiluminesenzdetektor hat den Vorteil, daß mit ihm gleichzeitig die Konzentration von NO und NO₂ ermittelt werden kann.

Da derartige Analysatoren sehr empfindlich auf Feuchtigkeit reagieren, was zu unerwünschten Nebeneffekten und Betriebsausfällen führen kann, ist erfindungsgemäß in der zum Analysator führenden Meßgasleitung eine Entfeuchteeinrichtung wie z.B. Kühler angeordnet, in welcher im Atemgas befindliche Feuchtigkeit, beispielsweise durch Auskondensieren, entzogen wird. Hierdurch wird erreicht, daß das Gerät über lange Zeiträume störungsfrei arbeitet. Desweiteren ist in der zum Analysator führenden Meßgasleitung eine Meßgaspumpe angeordnet. Diese zieht aus dem vom Respirator zum Patienten führenden Tubus eine größere Menge Atemluft ab, als der Analysator an sich benötigt. Die nicht benötigte Meßgasmenge wird über eine Bypassleitung an die Umgebung abgegeben. Hierdurch wird erreicht, daß Änderungen der Konzentration von NO und gegebenenfalls NO₂ sehr schnell vom Analysator erfaßt werden und die NO-Dosierung gegebenenfalls entsprechend geändert werden kann.

Zwei Ausführungsbeispiele der Erfindung sollen anhand der beigefügten Zeichnungen erläutert werden.

Es zeigen:
- Fig. 1: ein Gerät welches für beliebige Analysatoren zur Bestimmung von NO in der Atemluft eines Patienten geeignet ist,
- Fig. 2: ein Gerät mit einem Chemiluminesenzdetektor als Analysator, welches die gleichzeitige Ermittlung der Konzentrationen von NO und NO₂ gestattet.

Zu den Figuren sind gleiche Geräteteile mit gleichen Bezugszeichen versehen worden.

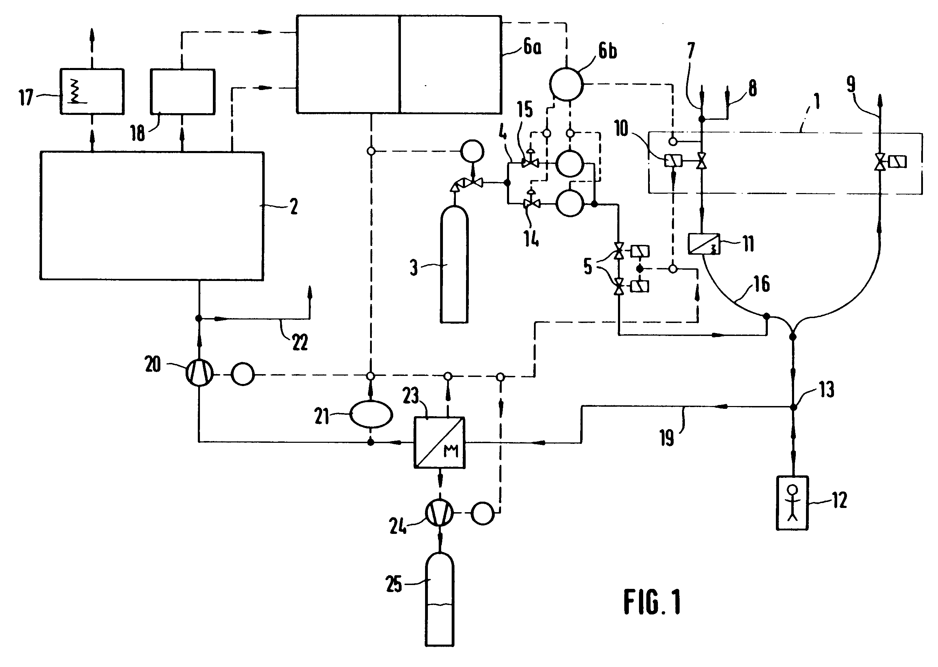

Die wesentlichen Bestandteile des in Figur 1 dargestellten Gerätes sind der Respirator 1, der Analysator 2, der NO-Dosierbehälter 3, die Dosiereinheit 4 mit Regelventilen 14, 15 und das Steuerungsgerät 6a. Die Steuer- und Regelverbindungen sind in der Zeichnung gestrichelt dargestellt, die Gasleitungen sind als ausgezogene Linien wiedergegeben. Diese Verbindungen sind überwiegend nicht mit Bezugszeichen versehen worden.

Der Respirator 1 ist das eigentliche Beatmungsgerät. Er besitzt Anschlüsse 7 und 8 für Atemluft und Sauerstoff, sowie einen Anschluß 9 zum Ableiten ausgeatmeter Luft. Die mit Sauerstoff angereicherte Luft durchströmt das Inspirationsventil 10 und den Anfeuchter 11. Der Patient 12 erhält somit über den Tubus 13 warme, stark mit Sauerstoff und Feuchtigkeit angereicherte Atemluft. Der NO-Dosierbehälter 3 ist eine Gasflasche und enthält ein Gemisch aus NO in N₂ 300-3000 vpm. Er ist an den Respirator 1, vorzugsweise an dessen respiratorischen Schenkel 16, angeschlossen. Der Analysator 2 ermittelt die Konzentration von NO. Die Steuerventile 5 können unter anderem vom Signal des Inspirationsventiles 10 mitgetaktet werden. Sollte dieses nicht zur Verfügung stehen, so regeln die Regelventile 14, 15 aufgrund des inspiratorischen Volumenstromes zu 0 und verschließen die NO-Zufuhr. Die Steuerventile 5 sind jedoch als Sicherheitsabschottungsventile in der NO-Zufuhr gedacht. Somit wird nur während der inspiratorischen Phase (Einatmen) der zudosierte NO-Gehalt analysiert während der exspiratorischen Phase (Ausatmen) jedoch das ausgeatmete Gasgemisch. Diese Anordnung ermöglicht es daher, das Analyseprodukt weiterzuinterpretieren und insbesondere das aufgenommene NO entsprechend zu deuten.

Die im Analysator 2 ermittelte Konzentration von NO wird kontinuierlich mit dem Registriergerät 17 aufgezeichnet. Die ermittelten Konzentrationswerte werden an das Steuerungsgerät 6a weitergeleitet, welches wiederum über die Dosiereinheit 4 mit den Regelventilen 14, 15 die dem Patienten zugeführte NO-Menge nachreguliert.

Durch Messung des durch die Anschlüsse 7 und 8 strömenden Volumenstroms von Atemluft und Sauerstoff und aufgrund der am Kontroll- und Regelgerät 6b eingestellten Konzentration errechnet das Kontroll- und Regelgerät 6b den erforderlichen Volumenstrom an zuzudosierendem NO. Dieser wird durch die automatische Ventileinstellung festgelegt. Anstelle eines Regelventiles können auch mehrere parallel geschaltete Ventile hierzu verwendet werden. Der in der NO-Dosierleitung gemessene NO-Volumenstrom wird als Regelgröße zurückgeführt. Aufgrund des Analysenwertes von NO regelt das Kontroll- und Regelgerät 6b die zu dosierte NO-Menge nach (doppelt vermaschter Regelkreis).

Dieses Regelkonzept ermöglicht es, nicht nur eine volumenskontrollierte Beatmung zu wählen, sondern auch sämtliche anderen Beatmungsformen zu praktizieren. Die NO-Konzentrationen werden hierbei immer konstant gehalten. Es ist daher auch die Beatmungsform der Spontanatmung denkbar, so daß auch nicht Vollintensivpatienten beatmet werden können.

Zusätzlich ist eine Grenzwertüberwachungseinheit 18 zwischen Analysator 2 und Steuerungsgerät 6a angeordnet. Diese schaltet die NO-Zufuhr zum Patienten ab, wenn im Tubus 13 eine für den Patienten gefährliche Konzentration von mehr als maximal 60 vpm NO im Atemgas gemessen wird. Eine Grenzwerteverschiebung ist jedoch möglich, indem der Grenzwert zwischen 0-60 vpm variabel einstellbar ausgebildet wird. Das Meßgas für den Analysator 2 wird aus dem Tubus 13 durch den Analyseabzug 19 entnommen. Der Analyseabzug 19 soll möglichst nahe am Patienten sein.

Erfindungsgemäß ist eine Meßgaspumpe 20 vorgesehen, welche in Verbindung mit einem Durchflußüberwachungsgerät 21 einen erheblich größeren Meßgasstrom aus dem Tubus 13 abzieht, als für den Betrieb des Analysators erforderlich ist. Die überschüssige Menge an Meßgas wird durch die Bypassleitung 22 abgeführt. Durch diese erfindungsgemäße Maßnahme wird erreicht, daß Änderungen der Konzentration von NO im Atemgas sehr schnell vom Analysator 2 ermittelt werden. Entsprechend schnell kann mittels des Steuerungsgerätes 6a und des Kontroll-und Regelgerätes 6b die NO-Zufuhr nachreguliert werden.

Patienten mit ARDS oder ähnlichem Erkrankungsbild müssen oft über Zeiträume von mehreren Wochen mit NO behandelt werden. Während dieser Behandlungsdauer soll das Gerät möglichst störungsfrei arbeiten. Um dies zu erreichen, ist nach einem weiteren Merkmal der Erfindung im Analyseabzug 19 ein Entfeuchter 23, vorzugsweise ein Meßgaskühler, angeordnet. Durch den Meßgaskühler wird die Feuchtigkeit aus dem Analysegasstrom weitgehend entfernt, ohne daß es zu irgendwelchen Reaktionen von NO und gegebenenfalls NO₂ kommt. Diese Feuchtigkeit könnte im Analysator 2 zu Nebeneffekten bis hin zum Betriebsausfall führen. Die im Meßgaskühler entfernte Feuchtigkeit wird mittels der Kondensatpumpe 24 in die Kondensatsammelflasche 25 geleitet und von Zeit zu Zeit entfernt.

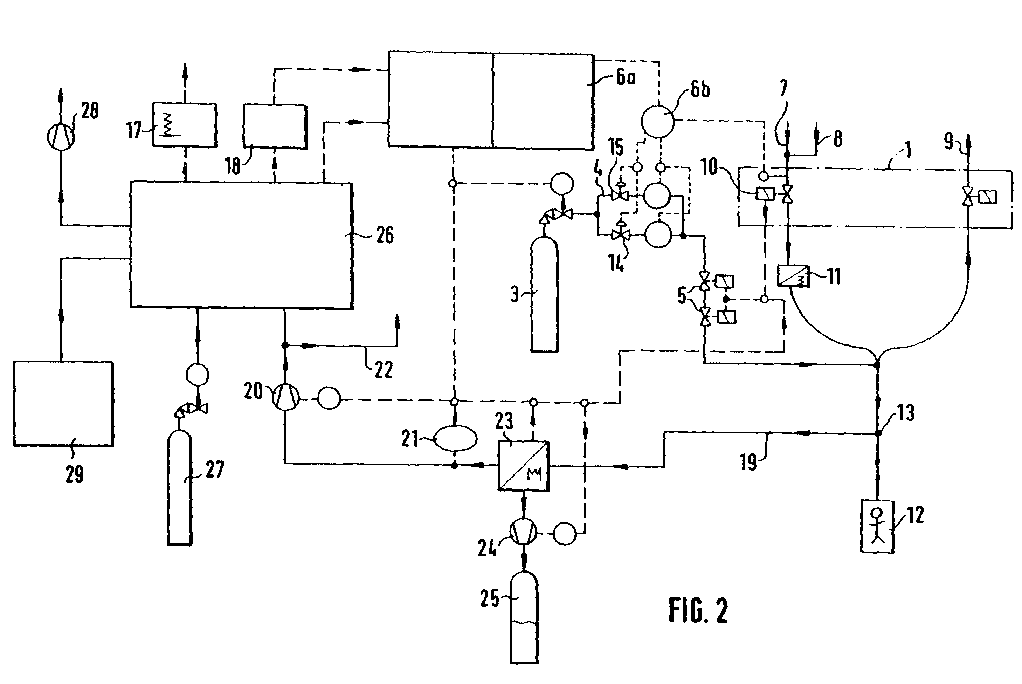

Bei dem in Figur 2 dargestellten Gerät ist der Analysator als Chemiluminesenzdetektor 26 ausgebildet, der es ermöglicht, gleichzeitig die Konzentrationen von NO und NO₂ zu ermitteln. Der Dosierbehälter 3 ist eine Gasflasche und enthält 900 vpm NO in N₂. Das giftige NO₂ entsteht in der Lunge des Patienten 12. Während der inspiratorischen Phase (Einatmen) wird der zudosierte NO- bzw. NO₂-Gehalt analysiert, während der exspiratorischen Phase (Ausatmen) das ausgeatmete Gasgemisch. Diese Anordnung ermöglicht es daher, das Analyseprodukt weiterzuinterpretieren und insbesondere aufgenommenes NO und entstandenes NO₂ entsprechend zu deuten. Neben anderen Faktoren ist die Menge des aus dem NO-Dosierbehälter 3 in den Tubus 13 eingespeisten NO maßgeblich für die Menge des gebildeten NO₂. Im Chemiluminesenzdetektor 26 reagiert NO mit Ozon, welches der Chemiluminesenzdetektor 26 selbst erzeugt. Bei dieser Reaktion entsteht Licht, dessen Intensität repräsentativ für die Konzentration des NO ist. Für den Betrieb des Chemiluminesenzdetektors 26 ist ein Prüfgas erforderlich, welches sich in der Prüfgasflasche 27 befindet. Es besteht aus 90 vpm NO in Stickstoff und dient zur Überprüfung bzw. Kalibrierung des Chemiluminesenzdetektors 26. Außerdem sind eine Vakuumpumpe 28 und ein Trockenluftgenerator 29 für den Betrieb des Chemiluminesenzdetektors 26 erforderlich. Die im Chemiluminesenzdetektor 26 ermittelten Konzentrationen von NO und NO₂ werden kontinuierlich mit dem Registriergerät 17 aufgezeichnet. Die ermittelten Konzentrationswerte werden an das Steuerungsgerät 6a weitergeleitet, welches wiederum über die Dosiereinheit 4 und die Steuerventile 5 die dem Patienten zugeführte NO-Menge nachreguliert.

Der Chemilumineszenzdetektor 26 arbeitet mit starkem Unterdruck, so daß an sich das Meßgas durch den Analyseabzug 19 selbsttätig zum Analysator 2 strömen würde. Die erfindungsgemäß vorgesehene Meßgaspumpe 20 zieht in Verbindung mit dem Durchflußüberwachungsgerät 21 einen erheblich größeren Meßgasstrom aus dem Tubus 13 ab, als für den Betrieb des Analysators erforderlich ist. Bei einem ausgeführten Gerät werden 3 l/min Meßgas abgezogen, während der Chemilumineszenzdetektor 26 nur 0,7 l/min benötigt. Die überschüssige Menge an Meßgas wird durch die Bypassleitung 22 abgeführt. Durch diese erfindungsgemäße Maßnahme wird erreicht, daß Änderungen der Konzentration von NO und NO₂ im Atemgas sehr schnell vom Analysator 2 ermittelt werden. Entsprechend schnell kann mittels des Steuerungsgerätes 6a und des Kontroll-und Regelgerätes 6b die NO-Zufuhr nachreguliert werden.

Durch die schnelle Änderung der NO-Dosierung aufgrund der ermittelten Konzentrationen von NO und NO₂ wird vorteilhafterweise erreicht, daß sich möglichst wenig an giftigen NO₂ bildet.

## Patentansprüche

1. Gerät zur kontrollierten Zudosierung von NO zur Atemluft von Patienten, bestehend aus
a) einem Respirator (1) mit einer vom Respirator zum Patienten führenden Verbindungsleitung (13),
b) einem an den Respirator (1) angeschlossenen NO-Dosierbehälter (3),
c) einer zwischen Respirator (1) und NO-Dosierbehälter (3) angeordneten Dosiereinheit (4),
d) einem an die vom Respirator (1) zum Patienten führende Verbindungsleitung (13) angeschlossenen Analysator (2) zur Bestimmung der Konzentration von NO in der Atemluft des Patienten,
e) einem an den Analysator (2) angeschlossenen und auf die Dosiereinheit (4) wirkenden Steuergerät (6a),
f) einem in der Verbindungsleitung zwischen Respirator (1) und Analysator (2) angeordneten Entfeuchter (23) und
g) einer in der Verbindungsleitung zwischen Analysator (2) und Entfeuchter (23) angeordneten Meßgaspumpe (20).

2. Gerät nach Anspruch 1, dadurch gekennzeichnet, daß der Entfeuchter (23) ein Meßgaskühler ist.

3. Gerät nach Anspruch 1 oder 2, dadurch gekennzeichnet, daß der Analysator (2) als nach dem Infrarot-Absorptionsmeßprinzip, als mit elektrochemischen Sensoren arbeitendes Gerät oder als Chemilumineszenzdetektor (26) ausgebildet ist.

4. Gerät nach einem der Ansprüche 1 bis 3, gekennzeichnet durch eine an den Analysator (2) angeschlossene und auf das Steuergerät (6a) wirkende NO-Grenzwertüberwachungseinheit (18).

5. Gerät nach einem der Ansprüche 1 bis 4, dadurch gekennzeichnet, daß die im Analysator (2) ermittelten NO-Konzentrationswerte an ein Steuergerät (6a) weitergeleitet, welches über eine Dosiereinheit (4) die dem Patienten zugeführte NO-Menge nachreguliert.

6. Gerät nach einem der Ansprüche 1 bis 5, gekennzeichnet durch Einrichtungen zum Erfassen des Inspirationsvolumensstroms aus Atemluft und Sauerstoff, des NO-Volumenstroms, sowie eine Kontroll- und Regeleinheit (6b), in welche der gemessene NO-Volumenstrom sowie der Analysenwert als Regelgröße zum Nachregeln der zudosierten NO-Menge zurückgeführt wird.

7. Gerät nach einem der Ansprüche 1 bis 6, dadurch gekennzeichnet, daß durch Messung des Volumenstroms von Atemluft und Sauerstoff und aufgrund der eingestellten NO-Konzentration ein Kontroll- und Regelgerät (6b) den erforderlichen Volumenstrom an zuzudosierendem NO errechnet.

8. Gerät nach Anspruch 3, dadurch gekennzeichnet, daß ein Chemilumineszenzdetektor (26) als Analysator (2) zur Bestimmung der Konzentration von NO und NO₂ im Atemgas oder ausgeatmeten Gas dient.

9. Gerät nach einem der Ansprüche 1 bis 8, dadurch gekennzeichnet, daß Steuerventile (5) in der NO zuführenden Leitung von einem Signal eines Inspirationsventiles (10) in der Leitung des respiratorischen Schenkels (16) mitgetaktet werden.

## Claims

1. Apparatus for the monitored metering of NO into patients' respiratory air, comprising
a) a respirator (1) with a connecting line (13) leading from the respirator to the patient,
b) an NO metering container (3) which is connected to the respirator (1),
c) a metering unit (4) arranged between respirator (1) and NO metering container (3),
d) an analyser (2) connected to the connecting line (13) leading from the respirator (1) to the patient for determining the concentration of NO in the patient's respiratory air,
e) a control unit (6a) which is connected to the analyser (2) and acts on the metering unit (4),
f) a dehumidifier (23) located in the connecting line between respirator (1) and analyser (2), and
g) a measurement gas pump (20) located in the connecting line between analyser (2) and dehumidifier (23).

2. Apparatus according to Claim 1, characterized in that the dehumidifier (23) is a measurement gas condenser.

3. Apparatus according to Claim 1 or 2, characterized in that the analyser (2) is designed as an apparatus operating on the principle of the measurement of infrared absorption or with electrochemical sensors, or as chemiluminescence detector (26).

4. Apparatus according to any of Claims 1 to 3, characterized by an NO limit monitoring unit (18) which is connected to the analyser (2) and acts on the control unit (6a).

5. Apparatus according to any of Claims 1 to 4, characterized in that the concentrations of NO measured in the analyser (2) [lacuna] transmitted to a control unit (6a) which adjusts, via a metering unit (4), the amount of NO supplied to the patient.

6. Apparatus according to any of claims 1 to 5, characterized by devices for measuring the inspiration volumetric flow of respiratory air and oxygen, the NO volumetric flow, and a monitoring and automatic control unit (6b) in which the measured NO volumetric flow and the analysed value is fed back as controlled variable for adjusting the amount of NO metered in.

7. Apparatus according to any of Claims 1 to 6, characterized in that a monitoring and automatic control unit (6b) calculates, by measuring the volumetric flow of respiratory air and oxygen and on the basis of the concentration of NO which is set, the required volumetric flow of NO to be metered in.

8. Apparatus according to Claim 3, characterized in that a chemiluminescence detector (26) serves as analyser (2) for determining the concentration of NO and NO₂ in the respiratory gas or exhaled gas.

9. Apparatus according to any of Claims 1 to 8, characterized in that control valves (5) in the line supplying NO are also clocked by a signal from an inspiration valve (10) in the line of the respiratory arm (16).

## Revendications

1. Appareil pour contrôler l'addition dosée de NO à l'air respiré par des patients, comprenant :
a) un respirateur (1) équipé d'une conduite de liaison (13) allant au patient,
b) un récipient de dosage de NO (3), raccordé au respirateur (1),
c) entre le respirateur (1) et le récipient (3), une unité de dosage (4),
d) un analyseur (2), raccordé à la conduite (13) allant du respirateur (1) au patient, et servant à déterminer la concentration en NO de l'air respiré par le patient,
e) un appareil de commande (6a) raccordé à l'analyseur (2) et agissant sur l'unité de dosage (4),
f) un déshumidificateur (23) monté dans la conduite reliant le respirateur (1) et l'analyseur (2),
g) une pompe de gaz de mesure (20) montée dans la conduite reliant l'analyseur (2) et le déshumidificateur (23).

2. Appareil selon la revendication 1,
caractérisé en ce que
le déshumidificateur (23) est un refroidisseur du gaz de mesure.

3. Appareil selon la revendication 1 ou 2,
caractérisé en ce que
l'analyseur (2) est un appareil fonctionnant selon le principe de mesure par absorption d'infrarouges, ou utilisant des détecteurs électrochimiques, ou constitué par un détecteur chimioluminescent (26).

4. Appareil selon l'une quelconque des revendications 1 à 3,
caractérisé en ce qu'
il comporte une unité de surveillance (18) de la valeur limite de NO, raccordé à l'analyseur (2) et agissant sur l'appareil de commande (6a) .

5. Appareil selon l'une quelconque des revendications 1 à 4,
caractérisé en ce que
les valeurs de concentration en NO établies dans l'analyseur (2) sont transmises à un appareil de contrôle (6a) qui, par l'intermédiaire d'une unité de dosage (4) réajuste la quantité de NO amenée au patient.

6. Appareil selon l'une quelconque des revendications 1 à 5,
caractérisé en ce qu'
il comporte des dispositifs de saisie du débit volumique inspiré, constitué d'air et d'oxygène, et du débit volumique de NO, ainsi qu'une unité de contrôle et de régulation (6b) à laquelle est renvoyée la mesure du débit volumique de NO ainsi que la valeur d'analyse, en tant que valeurs de régulation servant à réajuster la quantité du NO additionné de manière dosée.

7. Appareil selon l'une quelconque des revendications 1 à 6,
caractérisé en ce qu'
à partir de la mesure du débit volumique d'air à inspirer et d'oxygène, et en se basant sur la concentration en NO réglée, un appareil de contrôle et de régulation (6b) calcule le débit volumique nécessaire correspondant à l'addition dosée de NO.

8. Appareil selon la revendication 3,
caractérisé en ce qu'
un détecteur chimioluminescent (26) sert d'analyseur (2) pour déterminer les concentrations de NO et de NO₂ dans le gaz inspiré et dans le gaz expiré respectivement.

9. Appareil selon l'une quelconque des revendications 1 à 8,
caractérisé en ce que
des soupapes de commande (5), montées dans la canalisation amenant le NO, fonctionnent en synchronisme avec un signal d'une soupape d'inspiration (10) montée dans la conduite de la branche respiratoire (16).
